# EUROPEAN PATENT APPLICATION

(11) **EP 3 248 996 A1**
(43) Date of publication of application: **29.11.2017**
(21) Application number: 16740191.8
(22) Date of filing: 20.01.2016
(51) Int. Cl.: C08G 59/50, C08G 61/10, C08J 5/24

(54) **AROMATIC AMINE RESIN, AND EPOXY RESIN COMPOSITION AND CURED PRODUCT THEREOF**

(30) Priority: 21.01.2015 JP 2015009036
(71) Applicant: Nippon Kayaku Kabushiki Kaisha, Tokyo 100-0005 (JP)
(72) Inventor: KUBOKI Kenichi, Tokyo 115-8588 (JP); NAKANISHI Masataka, Tokyo 115-8588 (JP)
(74) Representative: Gille Hrabal
(86) International application number: PCT/JP2016/051516
(87) International publication number: WO 2016/117584

(57) **Abstract**

There are provided an aromatic amine resin represented by the following formula (1), which can be utilized for a high-reliability semiconductor, a high-performance fiber-reinforced composite material, and others; an epoxy resin composition containing the aromatic amine resin; and an epoxy resin cured product having properties excellent in high heat resistance and low hygroscopicity, which is obtained by curing the epoxy resin composition: 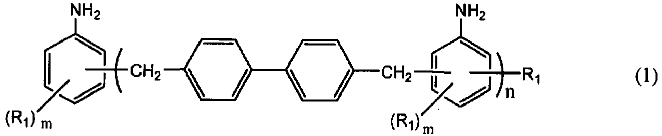 wherein a plurality of R₁'s each independently represent a hydrogen atom or an alkyl group having 1 to 4 carbon atoms, provided that a case where all R₁'s represent each a hydrogen atom is excluded, m represents an integer of 1 to 4, n represents an integer, and the average value (A) of n represents: 1 ≤ A ≤ 5.

## Description

### TECHNICAL FIELD

The present invention relates to an aromatic amine resin, an epoxy resin composition, a prepreg obtained by impregnating a reinforced fiber therewith, and a fiber-reinforced composite material containing a cured product of the epoxy resin composition as a constituent component. More specifically, it relates to an epoxy resin composition useful for electric/electronic component insulating materials including for high-reliability semiconductor encapsulation and for various composite materials including laminated sheets (printed wiring glass fiber-reinforced composite materials) and CFRP (carbon fiber-reinforced composite materials), for various adhesives, for various coatings, and for structural members and the like.

### BACKGROUND ART

Generally, in an epoxy resin composition to be used for fiber reinforced composite materials, an amine-based curing agent has been used and, particularly, for carbon fiber-reinforced composite materials, an aromatic amine-based curing agent has been often used. As the aromatic amine curing agent, diaminodiphenylmethane (DDM) and diaminodiphenyl sulfone (DDS) have been mainly used. On the other hand, dicyandiamide (DICY) has been often used in a glass fiber reinforced composite material for electric/electronic materials. These curing agents have broadly met the requirements of the production method of each composite material and the performance of the composite material and have been used for many years with adding various use methods, modifications, and the like. However, in recent years, since the use range and the use environment of the fiber-reinforced composite materials have rapidly extended, high performance has been required in electric properties, mechanical properties, and water resistance as compared to the performance of conventional one.

Patent Document 1: JP-B-1-259024
Patent Document 2: Japanese Patent No. 5019585

### DISCLOSURE OF THE INVENTION

### PROBLEMS TO BE SOLVED BY THE INVENTION

As mentioned above, in general, as aromatic amine-based curing agents for epoxy resins, diaminodiphenylmethane (DDM) and diaminodiphenyl sulfone (DDS) have been mainly used. Diaminodiphenylmethane is provided as crystals having a melting point of about 90°C and, since it also easily solves in a solvent, it is easy to mix it with an epoxy resin. However, since its reactivity with an epoxy group is good, there is a problem that the pot life of the epoxy resin composition is short. Diaminodiphenyl sulfone is provided as crystals having a melting point of about 175°C and, since it is inferior in reactivity to diaminodiphenylmethane, the pot life can be lengthened but, owing to its high melting point and its nature of difficulty in dissolution thereof in a solvent, it is difficult to mixed it with an epoxy resin. Moreover, dicyandiamide often used in glass fiber-reinforced composite materials for electric/electronic materials is excellent in potential of curing but the solubility in a solvent and an epoxy resin cannot be said to be good.

For structural materials for aircraft, automobiles, and the like, diaminodiphenylmethane or diaminodiphenyl sulfone has been often used. Particularly, in order to attain high heat resistance, diaminodiphenyl sulfone has been used but hygroscopicity increases and a decrease in physical properties after moisture absorption becomes a problem. In high reliability-requiring electronic components (ones for automotive, CPU, etc.) and the like, dicyandiamide has been often used and, also owing to the high hygroscopicity, a decrease in mechanical properties/electric properties after moisture absorption becomes a problem. Even in the epoxy resin composition using an aromatic amine resin shown in Patent Document 1, a cured product excellent in high heat resistance and impact resistance is obtained but it cannot be said that the product sufficiently meets the requirement in the decrease in the electric properties after moisture absorption. Moreover, in Patent Document 2, the decrease in the electric properties after moisture absorption is improved but the degree is not sufficient for satisfying the requirement.

### MEANS FOR SOLVING THE PROBLEMS

In consideration of such circumstances, the present inventors have intensively studied for seeking an aromatic amine resin and an epoxy resin composition capable of obtaining a cured product of the epoxy resin composition having high heat resistance and low hygroscopicity. As a result, they have accomplished the present invention.

That is, the present invention provides:
[1] An aromatic amine resin containing a compound represented by the following formula (1): 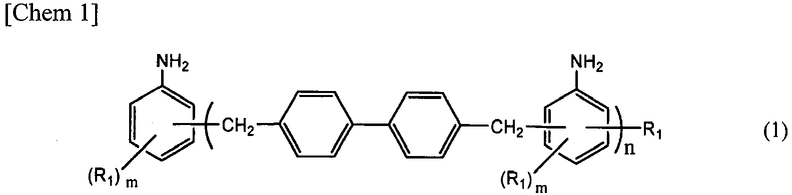 wherein a plurality of R₁'s each independently represent a hydrogen atom or an alkyl group having 1 to 4 carbon atoms, provided that a case where all R₁'s represent each a hydrogen atom is excluded, m represents an integer of 1 to 4, n represents an integer, and the average value (A) of n represents: 1 ≤ A ≤ 5.
[2] The aromatic amine resin as described in (1) above, wherein R₁'s in the formula (1) each independently represent a hydrogen atom or an alkyl group having 1 to 2 carbon atoms.
[3] The aromatic amine resin as described in (1) or (2) above, wherein the average value (A) of n in the formula (1) represents: 1 ≤ A ≤ 2.
[4] The aromatic amine resin as described in any one of (1) to (3) above, wherein R₁'s in the formula (1) are each an alkyl group and substitution positions thereof lie on 2-position and 6-position, on 2-position and 3-position, or on 2-position and 5-position with respect to the amino group.
[5] The aromatic amine resin as described in any one of (1) to (4) above, which contains the compound of n = 1 among the compounds represented by the formula (1) in an amount of 75% or more.
[6] An epoxy resin composition, which contains the aromatic amine resin as described in any one of (1) to (5) above.
[7] The epoxy resin composition as described in [6] above, which contains the epoxy resin represented by the following formula (2) and/or the following formula (3): 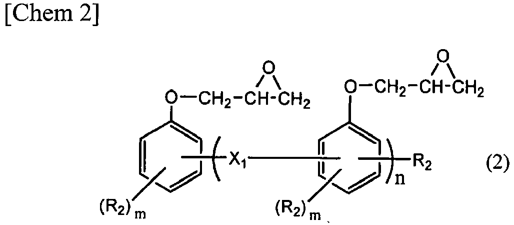 wherein a plurality of X₁'s each independently represent a hydrocarbon group having 5 to 20 carbon atoms or a hydrocarbon group having 5 to 20 carbon atoms and a heterocyclic ring, a plurality of R₂'s each independently represent a hydrogen, an alkyl group having 1 to 10 carbon atoms, a glycidyl ether group, or a phenyl group, m represents an integer of 1 to 4, n represents an integer, and the average value of n represents: 1 < n ≤ 10; 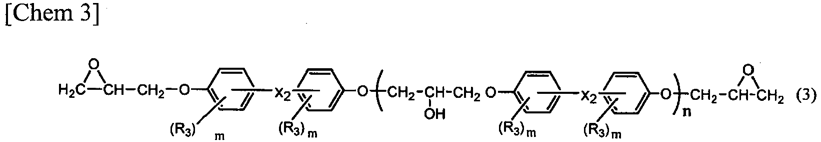 wherein a plurality of X₂'s each independently represent a single bond, a hydrocarbon group having 1 to 9 carbon atoms, a sulfur atom, an oxygen atom, -SO-, -SO₂-, -CO-,-CO₂-, or -Si(CH₃)₂-, R₃'s each independently represent a hydrogen, an alkyl group having 1 to 10 carbon atoms, a glycidyl ether group, or a phenyl group, m represents an integer of 1 to 4, n represents an integer, and the average value of n represents: 1 < n ≤ 10.
[8] The epoxy resin composition as described in [6] above, which contains an epoxy resin represented by the following formula (4): 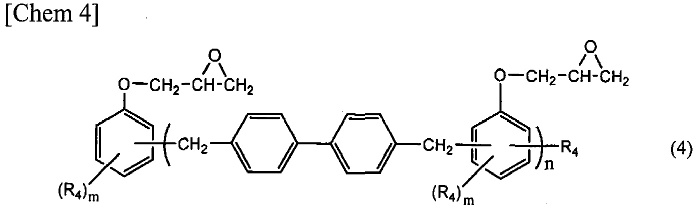 wherein a plurality of R₄'s each independently represent a hydrogen, an alkyl group having 1 to 10 carbon atoms, a glycidyl ether group, or a phenyl group, m represents an integer of 1 to 4, n represents an integer, and the average value of n represents: 1 < n ≤ 10.
[9] A prepreg obtained by impregnating a reinforced fiber with the epoxy resin composition as described in any one of [6] to [8] above.
[10] A cured product obtained by curing the epoxy resin composition as described in any one of [6] to [8] above.

### EFFECT OF THE INVENTION

The epoxy resin composition of the present invention using the aromatic amine resin of the present invention is wide in an adjustable range of the pot life and low in cure shrinkage and also has properties excellent in high heat resistance and low hygroscopicity in its cured product, so that the epoxy resin composition is useful for electric/electronic component insulating materials including for high-reliability semiconductor encapsulation and for various composite materials including laminated sheets (printed wiring glass fiber-reinforced composite materials) and CFRP (carbon fiber-reinforced composite materials), for various adhesives, for various coatings, and for structural members and the like..

### EMBODIMENTS FOR CARRYING OUT THE INVENTION

The following will describe the aromatic amine resin of the present invention.

The aromatic amine resin of the present invention contains a compound represented by the following formula (1). 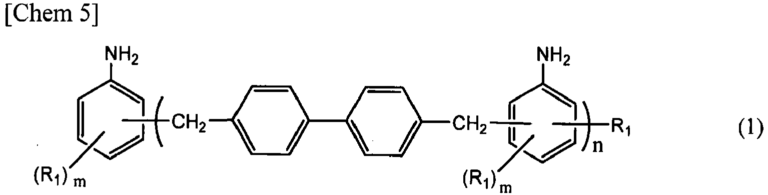 wherein a plurality of R₁'s each independently represent a hydrogen atom or an alkyl group having 1 to 4 carbon atoms, provided that a case where all R₁'s represent each a hydrogen atom is excluded, m represents an integer of 1 to 4, n represents an integer, and the average value (A) of n represents: 1 ≤ A ≤ 5.

R₁'s each independently are a hydrogen or an alkyl group having 1 to 4 carbon atoms and preferably a hydrogen or an alkyl group having 1 to 2 carbon atoms. However, a case in which all R₁'s represent each a hydrogen atom is excluded. It is because there is a concern of a decrease in heat resistance.

m is 1 to 4, preferably 1 to 2. The substitution position may lie anywhere but, in the case where m is 2, the substitution positions preferably lie on 2-position and 6-position, on 2-position and 3-position, or on 2-position and 5-position relative to the amino group.

As the alkyl group having 1 to 4 carbon atoms, for example, there may be mentioned a linear alkyl group having 1 to 4 carbon atoms. Specific examples thereof are a methyl group, an ethyl group, a propyl group, and a butyl group. R₁ is preferably a methyl group or an ethyl group.

n is, as an average value (A), as follows: 1 ≤ A ≤ 5, preferably 1 ≤ A ≤ 2, more preferably 1.1 ≤A≤2.

The following will describe a method for producing the aromatic amine resin of the present invention.

The aromatic amine resin of the present invention is obtained by reacting an aniline derivative having an alkyl group with a bishalogenomethylbiphenyl or a bisalkoxymethylbiphenyl in the presence of an acidic catalyst as required.

As the aniline derivative having an alkyl group to be used in the production of the compound of the formula (1), there may be mentioned 2-methylaniline, 3-methylaniline, 4-methylaniline, 2-ethylaniline, 3-ethylaniline, 4-ethylaniline , 2,3-dimethylaniline, 2,4-dimethylaniline, 2,5-dimethylaniline, 2,6-dimethylaniline, 3,4-dimethylaniline, 3,5-dimethylaniline, 2-propylaniline, 3-propylaniline, 4-propylaniline, 2-isopropylaniline, 3-isopropylaniline, 4-isopropylaniline, 2-ethyl-6-methylaniline, 2-sec-butylaniline, 2-tert-butylaniline, 4-butylaniline , 4-sec-butylaniline, 4-tert-butylaniline, 2,3-diethylaniline, 2,4-diethylaniline, 2,5-diethylaniline, 2,6-diethylaniline, 2-isopropyl-6-methylaniline, 4-aminobiphenyl, and the like. They may be used singly or in combination of two or more thereof.

Moreover, as the epoxy resin composition, from the viewpoint of capability of obtaining a cured product having more excellent heat resistance and low hygroscopicity, preferred are 2,3-dimethylaniline, 2,4-dimethylaniline, 2,5-dimethylaniline, 2, 6-dimethylaniline, 2,3-diethylaniline, 2,4-diethylaniline, 2,5-diethylaniline, and 2,6-diethylaniline, and particularly preferred are aniline derivatives having substituents at 2-position and 6-position, such as 2,6-dimethylaniline and 2,6-diethylaniline.

As the bishalogenomethylbiphenyl or bisalkoxymethylbiphenyl to be used, there may be mentioned 4,4'-bis(chloromethyl)biphenyl, 4,4'-bis(bromomethyl)biphenyl, 4,4'-bis(fluoromethyl)biphenyl, 4,4'-bis(iodomethyl)biphenyl, 4,4'-dimethoxymethylbiphenyl, 4,4'-diethoxymethylbiphenyl, 4,4'-dipropoxymethylbiphenyl, 4,4'-diisopropoxymethylbiphenyl, 4,4'-diisobutoxymethylbiphenyl, 4,4'-dibutoxymethylbiphenyl, 4,4'-di-tert-butoxymethylbiphenyl, and the like. They may be used singly or in combination of two or more thereof. Preferred are 4,4'-bis(chloromethyl)biphenyl, 4,4'-dimethoxymethylbiphenyl, and 4,4'-diethoxymethylbiphenyl, and particularly preferred is 4,4'-bis(chloromethyl)biphenyl.

The amount of the bishalogenomethylbiphenyl or bisalkoxymethylbiphenyl to be used is usually from 0.05 to 0.8 mol, preferably from 0.1 to 0.6 mol relative to 1 mol of the aniline to be used.

Examples of the acidic catalyst that can be used according to need include hydrochloric acid, phosphoric acid, sulfuric acid, formic acid, zinc chloride, ferric chloride, aluminum chloride, p-toluenesulfonic acid, methanesulfonic acid, and the like. They may be used singly or in combination of two or more thereof.

The amount of the catalyst to be used is from 0.1 to 0.8 mol, preferably 0.5 to 0.7 mol relative to 1 mol of the aniline to be used. When the amount is too large, the viscosity of the reaction solution is too high and stirring becomes difficult and, when the amount is too small, there is a concern that the progress of the reaction becomes slow.

The reaction may be carried out with using an organic solvent such as toluene or xylene as required or without any solvent. For example, after adding an acidic catalyst to a mixed solution of an aniline derivative and a solvent, in the case where the catalyst contains water, water is removed from the system in an azeotropic manner. Thereafter, the bishalogenomethylbiphenyl or the bisalkoxymethylbiphenyl is added thereto usually at 40 to 100°C, preferably 50 to 80°C over a period of 1 to 5 hours, preferably 2 to 4 hours, subsequently, while removing the solvent from the inside of the system, the temperature is raised, and the reaction is carried out at 180 to 240°c, preferably 190 to 220°C for 5 to 30 hours, preferably 10 to 20 hours. After completion of the reaction, the acidic catalyst is neutralized with an aqueous alkali solution, then a water-insoluble organic solvent is added to the oil layer, the whole is repeatedly washed with water until the waste water becomes neutral, and excess aniline derivative and the organic solvent are distilled off by heating under reduced pressure, thereby obtaining an aromatic amine resin of the above formula (1).

The amine equivalent of the aromatic amine resin of the present invention is preferably from 200 to 300 g/eq., particularly preferably from 200 to 240 g/eq.

The softening point of the aromatic amine resin of the present invention is preferably 180°C or lower, more preferably 150°C or lower. Further, the melt viscosity is preferably from 0.01 to 0.5Pa.s, particularly preferably from 0.01 to 0.15 Pa.s.

The following will describe the epoxy resin composition of the present invention.

As the epoxy resin which can be used in the epoxy resin composition of the present invention, specific examples thereof include glycidyl ether-based epoxy resins obtained by glycidylation of polycondensates of phenols (phenol, alkyl-substituted phenols, aromatic substituted phenols, naphthol, alkyl-substituted naphthols, dihydroxybenzenes, alkyl substituted dihydroxybenzenes, dihydroxynaphthalenes, etc.) with various aldehydes (formaldehyde, acetaldehyde, alkyl aldehydes, benzaldehyde, alkyl-substituted benzaldehydes, hydroxybenzaldehydes, naphthaldehyde, glutaraldehyde, phthalaldehyde, crotonaldehyde, cinnamaldehyde, etc.), polymers of phenols with various diene compounds (dicyclopentadiene, terpenes, vinylcyclohexene, norbornadiene, vinylnorbornene, tetrahydroindene, divinylbenzene, divinylbiphenyls, diisopropenylbiphenyls, butadiene, isoprene, etc.), polycondensates of phenols with ketones (acetone, methyl ethyl ketone, methyl isobutyl ketone, acetophenone, benzophenone, fluorenone, etc.), polycondensates of phenols with bishalogenomethylbenzenes or bishalogenomethylbiphenyls, polycondensates of bisphenols with various aldehydes, alcohols, and the like, alicyclic epoxy resins including 4-vinyl-1-cyclohexene diepoxide and 3,4-epoxycyclohexylmethyl-3,4'-epoxycyclohexane carboxylate as representatives, glycidyl amine-based epoxy resins including tetraglycidyldiaminodiphenylmethane (TGDDM) and triglycidyl-p-aminophenol as representatives, glycidyl ester-based epoxy resins, and the like but the epoxy resin is not limited thereto as long as it is an epoxy resin which is normally used. They may be used singly or in combination of two or more thereof. Preferred are epoxy resins represented by the following formulae (2) to (4). However, the epoxy resin is not limited thereto. 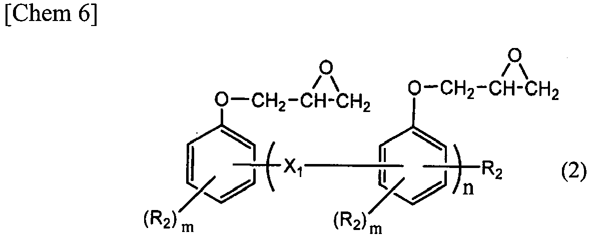 wherein a plurality of X₁'s each independently represent a hydrocarbon group having 5 to 20 carbon atoms or a hydrocarbon group having 5 to 20 carbon atoms and a heterocyclic ring, a plurality of R₂'s each independently represent a hydrogen, an alkyl group having 1 to 10 carbon atoms, a glycidyl ether group, or a phenyl group, m represents an integer of 1 to 4, n represents an integer, and the average value of n represents: 1 < n ≤ 10.

As the hydrocarbon group having 5 to 20 carbon atoms in X₁, there may be mentioned arylene groups such as a phenylene group, a phenylenebis(methylene) group, a methylphenylene group, a naphthalenediyl group, and an anthracenediyl group.

As the heterocyclic ring in the hydrocarbon group having 5 to 20 carbon atoms and having a heterocyclic ring in X₁, for example, there may be mentioned groups formed from a pyridine ring, a pyrazine ring, a pyrimidine ring, a pyridazine ring, a triazine ring, an indole ring, a quinoline ring, an acridine ring, a pyrrolidine ring, a dioxane ring, a piperidine ring, a morpholine ring, a piperazine ring, a carbazole ring, a furan ring, a thiophene ring, an oxazole ring, an oxadiazole ring, a benzoxazole ring, a thiazole ring, a thiadiazole ring, a benzothiazole ring, a triazole ring, an imidazole ring, a benzimidazole ring, a pyran ring, and a dibenzofuran ring, and the like.

As the alkyl group having 1 to 10 carbon atoms in R₂, for example, there may be mentioned a linear or branched alkyl group having 1 to 10 carbon atoms, and specific examples thereof include a methyl group, an ethyl group, a propyl group, a butyl group , an isobutyl group, a pentyl group, an isopentyl group, a neopentyl group, a hexyl group, an isohexyl group, a heptyl group, an isoheptyl group, an octyl group, a nonyl group, and a decyl group. Preferred is a methyl group, an ethyl group, a propyl group, a butyl group, or an isobutyl group, and more preferred is a methyl group. 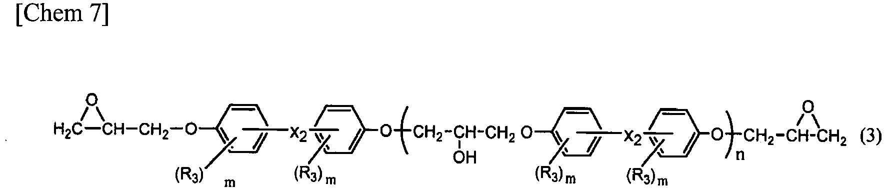 wherein a plurality of X₂'s each independently represent a single bond, a hydrocarbon group having 1 to 9 carbon atoms, a sulfur atom, an oxygen atom, -SO-, -SO₂-, -CO-,-CO₂-, or -Si(CH₃)₂-, R₃'s each independently represent a hydrogen, an alkyl group having 1 to 10 carbon atoms, a glycidyl ether group, or a phenyl group, m represents an integer of 1 to 4, n represents an integer, and the average value of n represents: 1 < n ≤ 10.

As the hydrocarbon group having 1 to 9 carbon atoms in X₂, there may be mentioned a methyl group, an ethyl group, a propyl group, an isopropyl group, a vinyl group, and a phenyl group.

As the alkyl group having 1 to 10 carbon atoms in R₃, for example, there may be mentioned a linear or branched alkyl group having 1 to 10 carbon atoms, and specific examples thereof include a methyl group, an ethyl group, a propyl group, a butyl group, an isobutyl group, a pentyl group, an isopentyl group, a neopentyl group, a hexyl group, an isohexyl group, a heptyl group, an isoheptyl group, an octyl group, a nonyl group, and a decyl group. Preferred is a methyl group, an ethyl group, a propyl group, a butyl group, or an isobutyl group, and more preferred is a methyl group. 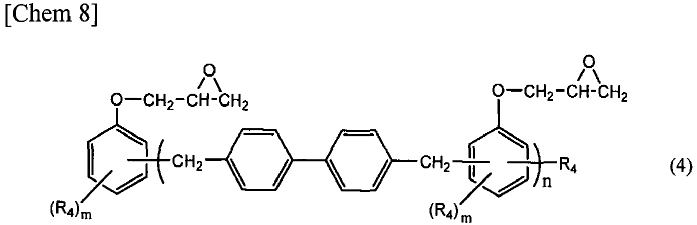 wherein a plurality of R₄'s each independently represent a hydrogen, an alkyl group having 1 to 10 carbon atoms, a glycidyl ether group, or a phenyl group, m represents an integer of 1 to 4, n represents an integer, and the average value of n represents: 1 < n ≤ 10.

As the alkyl group having 1 to 10 carbon atoms in R₄, for example, there may be mentioned a linear or branched alkyl group having 1 to 10 carbon atoms, and specific examples thereof include a methyl group, an ethyl group, a propyl group, a butyl group, an isobutyl group, a pentyl group, an isopentyl group, a neopentyl group, a hexyl group, an isohexyl group, a heptyl group, an isoheptyl group, an octyl group, a nonyl group, and a decyl group. Preferred is a methyl group, an ethyl group, a propyl group, a butyl group, or an isobutyl group, and more preferred is a methyl group.

In the epoxy resin composition of the present invention, the epoxy resins of the above formulae (2) to (4) can be used singly or in combination with another epoxy resin. In the case where epoxy resins are used in combination, the ratio of the epoxy resins of the formula (2) and/or the formula (3) and/or the formula (4) in the total epoxy resins is preferably 30% by weight or more, particularly preferably 40% by weight or more.

In the epoxy resin composition of the present invention, as the curing agent, the aromatic amine resin containing the compound represented by the above formula (1) can be used singly or in combination with another curing agent for an epoxy resin. In the case where curing agents are used in combination, the ratio of the aromatic amine resin containing the compound represented by the above formula (1) in the total curing agents is preferably 30% by weight or more, particularly preferably 40% by weight or more.

As the curing agent which may be used in combination with the aromatic amine resin containing the compound represented by the formula (1), for example, there may be mentioned amine-based compounds, acid anhydride-based compounds, amide-based compounds, phenol-based compounds, and the like. Specific examples of the curing agent which can be used include phenylenediamine, diaminodiphenylmethane, diaminodicyclohexylmethane, diaminodiphenyl ether, diethylenetriamine, triethylenetetramine, diaminodiphenyl sulfone, bisaniline M (manufactured by Mitsui Chemicals, Inc.), bisaniline P (manufactured by Mitsui Chemicals, Inc.), KAYAHARD A-A (manufactured by Nippon Kayaku Co., Ltd.), KAYABOND C Series (manufactured by Nippon Kayaku Co., Ltd.), isophoronediamine, norbornanediamine, dicyandiamide, a polyamide resin synthesized from a dimer of linolenic acid and ethylenediamine, phthalic anhydride, trimellitic anhydride, pyromellitic anhydride, maleic anhydride, tetrahydrophthalic anhydride, methyltetrahydrophthalic anhydride, methylnadic anhydride, hexahydrophthalic anhydride, methylhexahydrophthalic anhydride, phenol novolak, and modified products thereof, imidazole, BF3-amine complex, guanidine derivatives, and the like, but the curing agent is not limited thereto. They may be used singly or in combination of two or more thereof.

The amount of the curing agent to be used in the epoxy resin composition of the present invention is preferably from 0.7 to 1.2 equivalents relative to 1 equivalent of the epoxy group of the epoxy resin. In the case where the amount is less than 0.7 equivalents or more than 1.2 equivalents relative to 1 equivalent of the epoxy group, curing becomes incomplete and good curing properties may not be obtained in both cases.

Further, in the epoxy resin composition of the present invention, in the case where the aromatic amine resin containing the compound represented by the formula (1) as the curing agent, the gelation time can be also adjusted by using a curing accelerator.

Examples of the curing accelerator which can be used include imidazoles such as 2-methylimidazole, 2-ethylimidazole, and 2-ethyl-4-methylimidazole, tertiary amines such as 2-(dimethylaminomethyl)phenol and 1,8-diaza-bicyclo(5,4,0)undecene-7, phosphines such as triphenylphosphine, and metal compounds such as tin octylate. The curing accelerator is used in an amount of 0.02 to 5.0 parts by weight relative to 100 parts by weight of the epoxy resin according to need.

Furthermore, the epoxy resin composition of the present invention may be blended with a known additive as required.

Specific examples of the additive which can be used include polybutadiene and modified products thereof, modified products of acrylonitrile copolymers, polyphenylene ether, polystyrene, polyethylene, polyimides, fluorocarbon resins, maleimide-based compounds, cyanate ester-based compounds, silicone gels, silicone oils and inorganic fillers such as silica, alumina, calcium carbonate, quartz powder, aluminum powder, graphite, talc, clay, iron oxide, titanium oxide, aluminum nitride, asbestos, mica, and glass powder, surface treating agents of fillers, such as silane coupling agents, release agents, colorants such as carbon black, phthalocyanine blue, and phthalocyanine green.

The epoxy resin composition of the present invention may also be blended with a known maleimide-based compound as required.

Specific examples of the maleimide compound which may be used include 4,4'-diphenylmethanebismaleimide, polyphenylmethanemaleimide, m-phenylenebismaleimide, 2,2'-bis[4-(4-maleimidophenoxy)phenyl]propane, 3,3'-dimethyl-5,5'-diethyl-4,4'-diphenylmethanebismaleimide, 4-methyl-1,3-phenylenebismaleimide, 4,4'-diphenyl ether bismaleimide, 4,4'-diphenyl sulfone bismaleimide, 1,3-bis(3-maleimidophenoxy)benzene, 1,3-bis(4-maleimidophenoxy)benzene, and the like, but the compound is not limited thereto. They may be used singly or in combination of two or more thereof.

At the time of blending the maleimide-based compound, a curing accelerator is blended as required, and there may be mentioned the curing accelerator for the above epoxy resin, a radical polymerization initiator such as an organic peroxide or an azo compound, and the like.

The epoxy resin composition of the present invention can be obtained by mixing the individual components uniformly. The epoxy resin composition of the present invention can be easily converted into a cured product thereof by a method similar to a conventionally known method. For example, an epoxy resin of the present invention and a curing agent and, if necessary, a curing accelerator, an inorganic filler, and a blending agent are mixed well using an extruder, a kneader, a roll, or the like until they become homogeneous to obtain an epoxy resin composition and the epoxy resin composition is molded by casting after melting or using a transfer molding machine and further heated at 80 to 220°C for 2 to 10 hours. Thereby, a cured product can be obtained.

The epoxy resin composition of the present invention can be converted into a varnish-like composition (hereinafter, simply referred to as varnish) by adding an organic solvent to the resin composition.

As the solvent to be used, for example, there may be mentioned γ-butyrolactones, amide-based solvents such as N-methylpyrrolidone, N, N-dimethylformamide, N, N-dimethylacetamide, and N, N-dimethylimidazolidinone, sulfones such as tetramethylene sulfone, ether-based solvents such as diethylene glycol dimethyl ether, diethylene glycol diethyl ether, propylene glycol, propylene glycol monomethyl ether, propylene glycol monomethyl ether monoacetate, and propylene glycol monobutyl ether, ketone-based solvents such as methyl ethyl ketone, methyl isobutyl ketone, cyclopentanone, and cyclohexanone, and aromatic solvents such as toluene and xylene. The solvent is used in the range where the solid concentration excluding the solvent in the resulting varnish becomes usually from 10 to 80% by weight, preferably 20 to 70% by weight.

The prepreg of the present invention can be obtained by impregnating a reinforced fiber such as a glass fiber, a carbon fiber, a polyester fiber, a polyamide fiber, or an alumina fiber with the epoxy resin composition of the present invention after it is heated and melted to decrease its viscosity.

Also, the prepreg of the present invention can be obtained by impregnating a reinforced fiber with the varnish-like epoxy resin composition and then heating and drying the resultant.

After the prepreg is cut into a desired shape and laminated, a fiber-reinforced composite material can be obtained by heating and curing the epoxy resin composition while applying pressure to the laminate by a press molding method, an autoclave molding method, a sheet winding molding method, or the like.

In addition, at the time of lamination of the prepreg, it is also possible to laminate a copper foil or an organic film thereon.

### EXAMPLES

Hereinafter, the present invention will be specifically described by Examples and Comparative Examples. The present invention should not be construed as being limited to these Examples. In Synthetic Examples, Examples, and Comparative Examples, part(s) means part(s) by mass.

Incidentally, the amine equivalent, softening point, and ICI melt viscosity were measured under the following conditions.

### - Amine equivalent

It is measured by a method in accordance with the method described in JIS K-7236 Appendix A and a unit thereof is g/eq.

### - Softening point

It is measured by a method in accordance with JIS K-7234 and a unit thereof is °C.

### - ICI melt viscosity

It is measured by a method in accordance with JIS K 7117-2 and a unit thereof is Pa.s.

### Example 1

Into a flask fitted with a thermometer, a condenser, a Dean-Stark azeotropic distillation trap, and a stirrer were charged 428 parts of o-toluidine and 240 parts of toluene, and 63 parts of 35% hydrochloric acid was added dropwise at room temperature over a period of 1 hour. After completion of the dropwise addition, water and toluene in an azeotropic state were cooled and subjected to liquid separation and subsequently only toluene that was an organic layer was returned to the inside of the system, thereby achieving dehydration. Then, 126 parts of 4,4'-bis(chloromethyl)biphenyl was added over a period of 1 hour while the temperature was maintained at 60 to 70°C, and the reaction was further carried out at the same temperature for 2 hours. After completion of the reaction, toluene was distilled off while the temperature was raised, thereby heating the inside of the system to 205 to 210°C, and the reaction was carried out at this temperature for 12 hours. Thereafter, 223 parts of a 30% aqueous solution of sodium hydroxide was slowly added dropwise under cooling so as not to cause reflux violently, toluene having been distilled off at 80°C or lower was returned to the inside of the system, and the whole was allowed to stand at 70°C to 80°C. The separated lower aqueous layer was removed and the reaction solution was repeatedly washed with water until the wash liquid became neutral. Then, excess o-toluidine and toluene were distilled off from the oil layer by heating under reduced pressure, thereby obtaining 182 parts of an aromatic amine resin (A1) of the present invention. The softening point of the aromatic amine resin (A1) was 59°C, the melt viscosity was 0.04 Pa.s, and the amine equivalent was 203 g/eq.

### Example 2

Into a flask fitted with a thermometer, a condenser, a Dean-Stark azeotropic distillation trap, and a stirrer were charged 484 parts of 2,6-dimethylaniline and 240 parts of toluene, then 126 parts of 4,4'-bis(chloromethyl)biphenyl was added over a period of 1 hour while the temperature was maintained at 55 to 60°C, and the reaction was further carried out at 80 to 85°C for 1 hour and at 120°C for 1 hour. After completion of the reaction, toluene was distilled off while the temperature was raised, thereby heating the inside of the system to 207 to 210°C, and the reaction was carried out at this temperature for 5 hours. Thereafter, 139 parts of a 30% aqueous solution of sodium hydroxide was slowly added dropwise under cooling so as not to cause reflux violently, toluene having been distilled off at 80°C or lower was returned to the inside of the system, and the whole was allowed to stand at 70°C to 80°C. The separated lower aqueous layer was removed and the reaction solution was repeatedly washed with water until the wash liquid became neutral. Then, excess 2,6-dimethylaniline and toluene were distilled off from the oil layer by heating under reduced pressure, thereby obtaining 182 parts of an aromatic amine resin (A2) of the present invention. The softening point of the aromatic amine resin (A2) was 136°C, the melt viscosity was 0.04 Pa.s, and the amine equivalent was 211 g/eq.

### Example 3

Into a flask fitted with a thermometer, a condenser, a Dean-Stark azeotropic distillation trap, and a stirrer were charged 242 parts of 2,3-dimethylaniline and 120 parts of toluene, then 63 parts of 4,4'-bis(chloromethyl)biphenyl was added over a period of 1 hour while the temperature was maintained at 55 to 60°C, and the reaction was further carried out at 80 to 85°C for 2 hours and at 120°C for 1 hour. After completion of the reaction, toluene was distilled off while the temperature was raised, thereby heating the inside of the system to 205 to 210°C, and the reaction was carried out at this temperature for 24 hours. Thereafter, 72 parts of a 30% aqueous solution of sodium hydroxide was added dropwise under cooling, toluene having been distilled off at 80°C or lower was returned to the inside of the system, and the whole was allowed to stand at 70°C to 80°C. The separated lower aqueous layer was removed and the reaction solution was repeatedly washed with water until the wash liquid became neutral. Then, excess 2,3-dimethylaniline and toluene were distilled off from the oil layer by heating under reduced pressure, thereby obtaining 97 parts of an aromatic amine resin (A3) of the present invention. The softening point of the aromatic amine resin (A3) was 75°C, the melt viscosity was 0.09 Pa.s, and the amine equivalent was 217 g/eq.

### Example 4

Into a flask fitted with a thermometer, a condenser, a Dean-Stark azeotropic distillation trap, and a stirrer were charged 485 parts of 2,5-dimethylaniline and 240 parts of toluene, then 126 parts of 4,4'-bis(chloromethyl)biphenyl was added over a period of 1 hour while the temperature was maintained at 55 to 60°C, and the reaction was further carried out at 80 to 85°C for 1 hour and at 120°C for 1 hour. After completion of the reaction, toluene was distilled off while the temperature was raised, thereby heating the inside of the system to 207 to 210°C, and the reaction was carried out at this temperature for 30 hours. Thereafter, 143 parts of a 30% aqueous solution of sodium hydroxide was added dropwise under cooling, toluene having been distilled off at 80°C or lower was returned to the inside of the system, and the whole was allowed to stand at 70°C to 80°C. The separated lower aqueous layer was removed and the reaction solution was repeatedly washed with water until the wash liquid became neutral. Then, excess 2,5-dimethylaniline and toluene were distilled off from the oil layer by heating under reduced pressure, thereby obtaining 202 parts of an aromatic amine resin (A4) of the present invention. The softening point of the aromatic amine resin (A4) was 79°C, the melt viscosity was 0.14 Pa.s, and the amine equivalent was 217 g/eq.

### Example 5

Into a flask fitted with a thermometer, a condenser, a Dean-Stark azeotropic distillation trap, and a stirrer were charged 478 parts of 2,6-dimethylaniline and 200 parts of toluene, then 100 parts of 4,4'-bis(chloromethyl)biphenyl was added over a period of 1 hour while the temperature was maintained at 55 to 60°C, and the reaction was further carried out at 80 to 85°C for 1 hour and at 120°C for 1 hour. After completion of the reaction, toluene was distilled off while the temperature was raised, thereby heating the inside of the system to 207 to 210°C, and the reaction was carried out at this temperature for 14 hours. Thereafter, 119 parts of a 30% aqueous solution of sodium hydroxide was added dropwise under cooling, toluene having been distilled off at 80°C or lower was returned to the inside of the system, and the whole was allowed to stand at 70°C to 80°C. The separated lower aqueous layer was removed and the reaction solution was repeatedly washed with water until the wash liquid became neutral. Then, excess 2,6-diethylaniline and toluene were distilled off from the oil layer by heating under reduced pressure, thereby obtaining 182 parts of an aromatic amine resin (A5) of the present invention. The softening point of the aromatic amine resin (A5) was 115°C, the melt viscosity was 0.03 Pa.s, and the amine equivalent was 236 g/eq.

### Comparative Synthetic Example 1

Into a flask fitted with a thermometer, a condenser, a Dean-Stark azeotropic distillation trap, and a stirrer were charged 559 parts of aniline and 500 parts of toluene, and 167 parts of 35% hydrochloric acid was added dropwise at room temperature over a period of 1 hour. After completion of the dropwise addition, water and toluene in an azeotropic state were cooled and subjected to liquid separation and subsequently only toluene that was an organic layer was returned to the inside of the system, thereby achieving dehydration. Then, 251 parts of 4,4'-bis(chloromethyl)biphenyl was added over a period of 1 hour while the temperature was maintained at 60 to 70°C, and the reaction was carried out at the same temperature for 2 hours. After completion of the reaction, toluene was distilled off while the temperature was raised, thereby heating the inside of the system to 190 to 200°C, and the reaction was carried out at this temperature for 15 hours. Thereafter, 500 parts of a 30% aqueous solution of sodium hydroxide was slowly added dropwise under cooling so as not to cause reflux violently, toluene having been distilled off at 80°C or lower was returned to the inside of the system, and the whole was allowed to stand at 70°C to 80°C. The separated lower aqueous layer was removed and the reaction solution was repeatedly washed with water until the wash liquid became neutral. Then, excess aniline and toluene were distilled off from the oil layer by heating under reduced pressure, thereby obtaining 335 parts of an aromatic amine resin (C1). The softening point of the aromatic amine resin (C1) was 59°C, the melt viscosity was 0.05 Pa.s, and the amine equivalent was 196 g/eq.

### Examples 6 to 10

Each of the aromatic amine resins obtained in Examples 1 to 5 and an epoxy resin were blended in the ratio (parts by weight) shown in Table 1, and heated, melted, and mixed in a metal vessel, and the resultant was cast into a mold as it was and cured at 160°C for 2 hours and further at 180°C for 8 hours. Physical properties of the thus obtained cured products were measured for the following items and results thereof are shown in Table 1.
- Glass transition temperature: It was measured by TMA method (thermomechanical measurement apparatus: Q400EM manufactured by TA-instruments) at a temperature-raising rate of 2°C/min.
- Moisture absorption rate: Weight increase rate at 121°C/100% after 24 hours. A test specimen is a disk having a diameter of 50 mm and a thickness of 4 mm.
- Shrinkage factor: It was measured by a method in accordance with JIS K-6911 (molding shrinkage factor)

### Comparative Examples 1 and 2

Using the aromatic amine resin (C1) obtained in Comparative Synthetic Example 1 or diaminodiphenylmethane (DDM) and an epoxy resin, a resin molded body was prepared in the same manner as in Examples, and physical properties of cured products were measured. The results are shown in Table 1.
[Table 1]

**Table 1**

| | Example 6 | Example 7 | Example 8 | Example 9 | Example 10 | Comparative Example 1 | Comparative Example 2 |
|---|---|---|---|---|---|---|---|
| Epoxy resin | E1 | E1 | E1 | E1 | E1 | E1 | E1 |
| | 100 | 100 | 100 | 100 | 100 | 100 | 100 |
| Curing agent | A1 | A2 | A3 | A4 | A5 | C1 | DDM |
| | 55.2 | 57.3 | 59 | 59 | 64.1 | 53.3 | 26.9 |
| Glass transition temperature (°C) | 185 | 195 | 191 | 195 | 192 | 166 | 173 |
| Moisture absorption rate (%) | 1.7 | 1.7 | 1.6 | 1.6 | 1.7 | 1.8 | 2.4 |
| Shrinkage factor (%) | 1.0 | 0.9 | 1.0 | 0.9 | 0.8 | 1.7 | 1.2 |

| | | | | | | | |
|---|---|---|---|---|---|---|---|
| Notes) (E1): RE-310S (manufactured by Nippon Kayaku Co., Ltd., epoxy equivalent: 184 g/eq) (A1): Aromatic amine resin synthesized in Example 1 (A2): Aromatic amine resin synthesized in Example 2 (A3): Aromatic amine resin synthesized in Example 3 (A4): Aromatic amine resin synthesized in Example 4 (A5): Aromatic amine resin synthesized in Example 5 (C1): Aromatic amine resin synthesized in Comparative Synthetic Example 1 (DDM): Diaminodiphenylmethane | | | | | | | |

### Examples 11 to 14 and Comparative Examples 3 and 4

Each of the aromatic amine resins obtained in Examples 1 to 5 and Comparative Synthetic Example 1 was used and each of various epoxy resins and a curing accelerator were blended thereto in the ratio (parts by weight) shown in Table 2. After the blend was kneaded in a mixing roll and formed into tablets, a resin molded body was prepared by transfer molding and cured at 160°C for 2 hours and further at 180°C for 6 hours. Physical properties of the thus obtained cured products were measured for the following items and results thereof are shown in Table 2.
[Table 2]

**Table 2**

| | Example 11 | Example 12 | Comparative Example 3 | Example 13 | Example 14 | Comparative Example 4 |
|---|---|---|---|---|---|---|
| Epoxy resin | E2 | E2 | E2 | E3 | E3 | E3 |
| | 100 | 100 | 100 | 100 | 100 | 100 |
| Curing agent | A1 | A2 | C1 | A1 | A2 | C1 |
| | 36.6 | 38 | 35.2 | 51.3 | 53.2 | 50 |
| Curing accelerator | Salicylic acid 1.7 | Salicylic acid 2 | Salicylic acid 1.5 | Salicylic acid 1.3 | Salicylic acid 1 | Salicylic acid 0.8 |
| Glass transition temperature (°C) | 189 | 201 | 165 | 202 | 209 | 191 |
| Moisture absorption rate (%) | 0.9 | 0.9 | 1.1 | 1.3 | 1.4 | 1.5 |
| Shrinkage factor (%) | 1.3 | 1.1 | 1.7 | 1.4 | 1.2 | 1.8 |

| | | | | | | |
|---|---|---|---|---|---|---|
| Notes) (E2): EOCN-1020-70 (manufactured by Nippon Kayaku Co., Ltd., epoxy equivalent: 198 g/eq) (E3): NC-3000 (manufactured by Nippon Kayaku Co., Ltd., epoxy equivalent: 277 g/eq) | | | | | | |

From Tables 1 and 2, it can be confirmed that the cured products of the epoxy resin compositions using the aromatic amine resins of the present invention have improved heat resistance and also have improved hygroscopicity and shrinking properties as compared with the cured products of the epoxy resin compositions using the aromatic amine resins for comparison and diaminodiphenylmethane generally used as a curing agent.

While the present invention has been described in detail and with reference to specific embodiments thereof, it will be apparent to one skilled in the art that various changes and modifications can be made therein without departing from the spirit and scope thereof.

The present application is based on Japanese Patent Application No. 2015-009036 filed on January 21, 2015, and the entire contents are incorporated herein by reference. Also, all the references cited herein are incorporated as a whole.

### INDUSTRIAL APPLICABILITY

According to the aromatic amine resin and the epoxy resin composition of the present invention, a cured product of the epoxy resin composition having high heat resistance and low hygroscopicity can be obtained and thus can be utilized for fiber-reinforced composite materials.

## Claims

1. An aromatic amine resin containing a compound represented by the following formula (1): 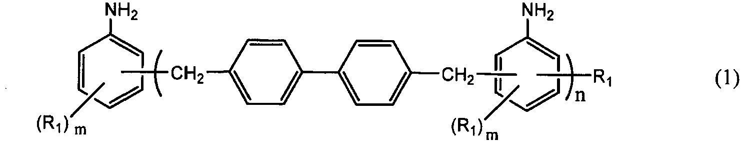 wherein a plurality of R₁'s each independently represent a hydrogen atom or an alkyl group having 1 to 4 carbon atoms, provided that a case where all R₁'s represent each a hydrogen atom is excluded, m represents an integer of 1 to 4, n represents an integer, and the average value (A) of n represents: 1 ≤ A ≤ 5.

2. The aromatic amine resin according to claim 1, wherein R₁'s in the formula (1) each independently represent a hydrogen atom or an alkyl group having 1 to 2 carbon atoms.

3. The aromatic amine resin according to claim 1 or 2, wherein the average value (A) of n in the formula (1) represents: 1 ≤ A ≤ 2.

4. The aromatic amine resin according to any one of claims 1 to 3, wherein R₁'s in the formula (1) are each an alkyl group and substitution positions thereof lie on 2-position and 6-position, on 2-position and 3-position, or on 2-position and 5-position with respect to the amino group.

5. The aromatic amine resin according to any one of claims 1 to 4, which contains the compound of n = 1 among the compounds represented by the formula (1) in an amount of 75% or more.

6. An epoxy resin composition, which contains the aromatic amine resin according to any one of claims 1 to 5.

7. The epoxy resin composition according claim 6, which contains the epoxy resin represented by the following formula (2) and/or the following formula (3): 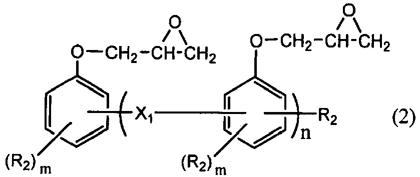 wherein a plurality of X₁'s each independently represent a hydrocarbon group having 5 to 20 carbon atoms or a hydrocarbon group having 5 to 20 carbon atoms and a heterocyclic ring, a plurality of R₂'s each independently represent a hydrogen, an alkyl group having 1 to 10 carbon atoms, a glycidyl ether group, or a phenyl group, m represents an integer of 1 to 4, n represents an integer, and the average value of n represents: 1 < n ≤ 10; 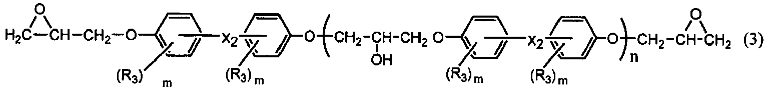 wherein a plurality of X₂'s each independently represent a single bond, a hydrocarbon group having 1 to 9 carbon atoms, a sulfur atom, an oxygen atom, -SO-, -SO₂-, -CO-,-CO₂-, or -Si(CH₃)₂-, R₃'s each independently represent a hydrogen, an alkyl group having 1 to 10 carbon atoms, a glycidyl ether group, or a phenyl group, m represents an integer of 1 to 4, n represents an integer, and the average value of n represents: 1 < n ≤ 10.

8. The epoxy resin composition according to claim 6, which contains an epoxy resin represented by the following formula (4): 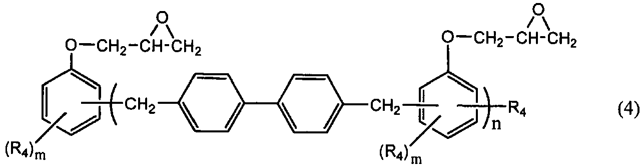 wherein a plurality of R₄'s each independently represent a hydrogen, an alkyl group having 1 to 10 carbon atoms, a glycidyl ether group, or a phenyl group, m represents an integer of 1 to 4, n represents an integer, and the average value of n represents: 1 < n ≤ 10.

9. A prepreg obtained by impregnating a reinforced fiber with the epoxy resin composition according to any one of claims 6 to 8.

10. A cured product obtained by curing the epoxy resin composition according to any one of claims 6 to 8.
